# EUROPEAN PATENT APPLICATION

(11) **EP 4 613 862 A1**
(43) Date of publication of application: **10.09.2025**
(21) Application number: 23884775.0
(22) Date of filing: 27.10.2023
(51) Int. Cl.: C12N 15/29, C12N 15/82, C12N 1/15, C12N 5/10, C12N 1/21, C12N 15/11, C12N 1/19, A01H 5/00, C07K 14/415

(54) **DISEASE RESISTANCE-RELATED PROTEIN RCR1, CODING GENE THEREOF, AND USE THEREOF**

(30) Priority: 02.11.2022 CN 202211360611
(71) Applicant: INSTITUTE OF GENETICS AND DEVELOPMENTAL BIOLOGY, CHINESE ACADEMY OF SCIENCES, Chaoyang District Beijing 100101 (CN)
(72) Inventor: ZHOU, Jianmin, Beijing 100101 (CN); WANG, Wei, Beijing 100101 (CN); ZHANG, Wenjing, Beijing 100101 (CN); HUANG, Yun, Beijing 100101 (CN); ZHANG, Chao, Beijing 100101 (CN)
(74) Representative: BASF IP Association
(86) International application number: PCT/CN2023/127263
(87) International publication number: WO 2024/093843

(57) **Abstract**

The present invention disclosed a disease resistance-related protein RCRl, a coding gene thereof, and use thereof. The disease resistance-related protein RCR1 provided by the present invention is a protein set forth in Sequence 2. The present invention demonstrates by means of experiments that when RCR1-transgenic *Arabidopsis thaliana* or *Brassica napus* is infected with *Plasmodiophora brassicae,* the resistance to *Plasmodiophora brassicae* is significantly enhanced as compared with the wild-type *Arabidopsis thaliana* or *Brassica napus*; and the resistance to *Plasmodiophora brassicae* of *RCR1* gene-knock out *Arabidopsis thaliana* is significantly reduced compared with the wild-type *Arabidopsis thaliana.* It is indicated that the *RCRl* gene is related to the resistance of *Arabidopsis thaliana* to *Plasmodiophora brassicae,* and the RCR1 protein and the coding gene thereof can be used for cultivating clubroot-resistant varieties in agricultual production. The present invention is of great significance for breeding disease-resistant plant varieties, especially for breeding new varieties of clubroot-resistant Brassicaceae crops, and can be used for breeding and identifying disease-resistant plant varieties required for agricultual production.

## Description

### TECHNICAL FIELD

The present invention belongs to the field of plant genetic engineering, and specifically relates to a disease resistance-related protein RCR1, an encoding gene, and use thereof.

### BACKGROUND OF THE INVENTION

Brassicaceae plants, including *Brassica napus*(*B. napus*), are crucial sources of oilseeds, In recent years, clubroot, a disease caused by *Plasmodiophora brassicae*(*P. brassicae*) in cruciferous oilseed crops, has significantly impacted the yield and quality of these crops.

The model plant *Arabidopsis thaliana*(*A.thaliana*) also belongs to the Brassicaceae family. As research into the interaction mechanism between *A*. *thaliana* and *P. brassicae* continues to deepen, utilizing disease-resistant genes for breeding resistant varieties has emerged as the most effective, safe, and economical strategy for experts to manage clubroot in crops such as *B. napus.*

### SUMMARY OF THE INVENTION

The technical problem to be solved by the present invention is how to regulate plant disease resistance and cultivate a disease-resistant plant variety.

In a first aspect, the present invention provides a DNA molecule.

The DNA molecule provided by the present invention comprises, in order from the 5' end, a transcription regulatory element of *RCR1* gene and a *RCR1* gene;
wherein the transcription regulatory element of *RCR1* gene is as follows A1) or A2):
A1) a DNA molecule as shown at positions 1-3643 of Sequence 3;
A2) a DNA molecule having 50% or higher than 50% identity to the nucleotide sequence defined in A1) and having transcriptional regulatory function;
and the *RCR1* gene is as follows B1) or B2):
   B1) a DNA molecule as shown at positions 3644-4090 of Sequence 3;
   B2) a DNA molecule having 50% or higher than 50% identity to the nucleotide sequence defined in B1) and having the same function as the nucleotide sequence defined in B1).

Further, the DNA molecule comprises, in order from the 5' end, the transcription regulatory element of *RCR1* gene, the *RCR1* gene, and a 3 'UTR region

The nucleotide sequence of the 3 'UTR region is as follows C1) or C2):
C1) a DNA molecule as shown at positions 4091-4800 of Sequence 3;
C2) a DNA molecule having 50% or higher than 50% identity to the nucleotide sequence defined in C1) and having the same function as the nucleotide sequence defined in C1).

Furthermore, the DNA molecule is composed of the transcription regulatory element of *RCR1* gene, the *RCR1* gene, and the 3 'UTR region in order from the 5' end.

The nucleotide sequence of the DNA molecule is as follows D1) or D2):
D1) a DNA molecule of Sequence 3;
D2) a DNA molecule having 50% or higher than 50% identity to the nucleotide sequence defined in D1) and having the same function as the nucleotide sequence defined in D1).

In A2) or B2) or C2) or D2) mentioned above, the identity includes a nucleotide sequence that has 50% or higher, or 55% or higher, or 60% or higher, or 65% or higher, or 70% or higher, or 75% or higher, or 80% or higher, or 85% or higher, or 90% or higher, or 95% or higher identity with the nucleotide sequence shown at positions 1-3643 of Sequence 3 of the present invention, or with the nucleotide sequence shown at positions 3644-4090 of Sequence 3 of the present invention, or with the nucleotide sequence shown at positions 4091-4800 of Sequence 3 of the present invention, or with the nucleotide sequence of Sequence 3 of the present invention. Identity can be evaluated with the naked eye or computer software. Using computer software, the identity between two or more sequences can be expressed as a percentage (%), which can be used to evaluate the identity between related sequences.

In a second aspect, the present invention provides a biological material related to the aforementioned DNA molecule.

The biological material related to the aforementioned DNA molecule provided by the present invention is a recombinant vector containing the above-mentioned DNA molecule, a recombinant microorganism containing the above-mentioned DNA molecule, or a transgenic plant cell line containing the above-mentioned DNA molecule.

The vector may be a plasmid, a cosmid, a phage, or a viral vector. The recombinant vector may be a vector containing the above-mentioned DNA molecule.

The microorganism may be yeast, bacteria, algae or fungi, such as *Agrobacterium tumefaciens*(*A. tumefaciens*)*.* The recombinant microorganism may be a microorganism containing the aforementioned DNA molecule or the aforementioned recombinant vector.

In a third aspect, the present invention provides a new use of the aforementioned DNA molecule or biological material related to the aforementioned DNA molecule.

The present invention provides a use of the aforementioned DNA molecule or the biological material related to the aforementioned DNA molecule in the following M1) or M2):
M1) regulating plant disease resistance;
M2) cultivating a transgenic plant or gene-edited plant with enhanced disease resistance.

In the above use, the regulating plant disease resistance is to improve plant disease resistance; the enhanced disease resistance is embodied in: a susceptible plant containing the aforementioned DNA molecule become disease-resistant; further embodied in: a plant susceptible to clubroot containing the above DNA molecule shows a clubroot resistance.

In a fourth aspect, the present invention provides a new use of the plant disease resistance-related protein RCR1.

The present invention provides use of RCR1 protein in following M1) or M2):
M1) regulating plant disease resistance;
M2) cultivating a transgenic plant or gene-edited plant with enhanced disease resistance; and the RCR1 protein is N1) or N2) or N3) or N4):
N1) a protein having an amino acid sequence of Sequence 2;
N2) a fusion protein obtained by attaching a tag to the N-terminus and/or C-terminus of the protein of Sequence 2;
N3) a plant disease resistance related protein obtained from the amino acid sequence of Sequence 2 by substitution, and/or deletion, and/or addition of one or more amino acid residues;
N4) a plant disease resistance related protein having 75% identity to the amino acid of Sequence 2.

Among them, Sequence 2 consists of 148 amino acid residues.

Among the protein described in N2) above, the tag refers to a polypeptide or protein that is fused and expressed together with the target protein using DNA in vitro recombination technology to facilitate the expression, detection, tracing and/or purification of the target protein. The tag may be a Flag tag, His tag, MBP tag, HA tag, myc tag, GST tag and/or SUMO tag, etc.

In the protein described in N3) above, the substitution and/or deletion and/or addition of one or more amino acid residues is a substitution and/or deletion and/or addition of no more than 35 amino acid residues, or no more than 30 substitution and/or deletion and/or addition of amino acid residues, or no more than 25 substitutions and/or deletions and/or additions of amino acid residues, or no more than 20 substitutions and/or deletions and/or additions of amino acid residues, or no more than 15 substitutions and/or deletions and/or additions of amino acid residues, or no more than 10 substitutions and/or deletions and/or additions of amino acid residues, or no more than 9 substitutions and/or deletions and/or additions of amino acid residues, or no more than 8 substitutions and/or deletions and/or additions of amino acid residues, or no more than 7 substitutions and/or deletions and/or additions of amino acid residues, or no more than 6 substitutions and/or deletions and/or additions of amino acid residues, or no more than 5 substitutions and/or deletions and/or additions of amino acid residues, or no more than 4 substitutions and/or deletions and/or additions of amino acid residues, or no more than 3 substitutions and/or deletions and/or additions of amino acid residues, or no more than 2 substitutions and/or deletions and/or additions of amino acid residues, or no more than 1 substitutions and/or deletions and/or additions of amino acid residues.

In the protein described in N4) above, "identity" includes a amino acid sequence having75% or higher, or 80% or higher, or 85% or higher, or 90% or higher, or 91% or higher, or 92% or higher, or 93 % or higher, or 94% or higher, or 95% or higher, or 96% or higher, or 97% or higher, or 98% or higher, or 99% or higher identity to the amino acid sequence of Sequence 2.

The protein described in the above N1) or N2) or N3) or N4) can be artificially synthesized, or obtained by firstly encoding its coding gene can be synthesized first and then biologically expressed.

In a fifth aspect, the present invention provides a new use of a biological material related to RCR1 protein.

The present invention provides the use of the biological material related to RCR1 protein in the following M1) or M2):
M1) regulating plant disease resistance;
M2) cultivating a transgenic plant or gene-edited plant with enhanced disease resistance;
the biological material related to RCR1 protein is a nucleic acid molecule encoding the RCR1 protein, or an expression cassette comprising the nucleic acid molecule, a recombinant vector comprising the nucleic acid molecule, a recombinant microorganism comprising the nucleic acid molecule, or a transgenic plant cell line comprising the nucleic acid molecule.

In the above use, the nucleic acid molecule encoding the RCR1 protein is a DNA molecule represented by the following P1) or P2) or P3):
P1) a DNA molecule shown in Sequence 1;
P2) a DNA molecule shown at positions 3644-4090 of Sequence 3;
P3) a DNA molecule that has 50% or more identity to the nucleotide sequence defined by P1) or P2) and encodes the RCR1 protein.

Among them, the nucleic acid molecule can be DNA, such as cDNA, genomic DNA or recombinant DNA; the nucleic acid molecule can also be RNA, such as mRNA or hnRNA, etc.

Those of ordinary skill in the art can easily mutate the nucleotide sequence encoding the RCR1 protein of the present invention using known methods, such as directed evolution and point mutation. Those artificially modified nucleotides that have 50% or higher identity with the nucleotide sequence encoding the RCR1 protein, as long as they encode the RCR1 protein and have the same function, are derived from the nucleotide sequence of the present invention and are equivalent to Sequences of the invention.

In the above P3), the identity includes a nucleotide sequence having 50% or higher, or 55% or higher, or 60% or higher, or 65% or higher, or having 70% or higher, or having 75% or higher, or having 80% or higher, or having 85% or higher, or having 90% or higher, or 95% or higher identity to the nucleotide sequence of the protein consisting of the amino acid sequence shown in the coding Sequence 2 of the present invention. Identity can be assessed with the naked eye or with computer software. Using computer software, the identity between two or more sequences can be expressed as a percentage (%), which can be used to evaluate the identity between related sequences.

The expression cassette refers to DNA capable of expressing RCR1 protein in a host cell. The DNA may not only include a promoter that initiates *RCR1* transcription, but may also include a terminator that terminates *RCR1* transcription. Furthermore, the expression cassette may also include an enhancer sequence. Promoters useful in the present invention include, but are not limited to: constitutive promoters; tissue, organ and development specific promoters and inducible promoters. Suitable transcription terminators include, but are not limited to: terminator of *A*. *tumefaciens* nopaline synthase (NOS terminator), cauliflower mosaic virus CaMV 35S terminator, *tml* terminator, pea rbcS E9 terminator and nopaline and octopine synthase terminator. In a specific embodiment of the present invention, the expression cassette can be the DNA molecule shown in Sequence 3. In another specific embodiment of the present invention, the expression cassette can be the DNA molecule shown in Sequence 5.

The vector may be a plasmid, cosmid, phage or viral vector. The recombinant vector may be a vector containing the *RCR1* gene expression cassette constructed using an existing plant expression vector. The plant expression vectors include binary A. *tumefaciens* vectors and vectors that can be used for plant gene gun transformation, etc, such as pAHC25, pBin438, pCAMBIA1302, pCAMBIA2301, pCAMBIA1301, pCAMBIA1300, pBI121, pCAMBIA1391-Xa or pCAMBIA1391-Xb, etc. The plant expression vector may also contain the 3' untranslated region of the foreign gene, that is, containing the poly(A) signal and any other DNA fragments involved in mRNA processing or gene expression. The poly(A) signal can guide poly(A) to be added to the 3' end of the mRNA precursor. For example, the untranslated regions transcribed at the 3' end of storage protein genes of the *A*. *tumefaciens* crown gall tumor induction (Ti) plasmid gene (such as nopaline synthase gene *Nos*), plant genes (such as soybean) all have similar functions. When using the gene of the present invention to construct a plant expression vector, enhancers including translation enhancers or transcription enhancers can also be used. These enhancer regions can be ATG start codons or adjacent region start codons, but must be the same as the reading frame of the coding sequence to ensure correct translation of the entire sequence. The translation control signals and initiation codons come from a wide range of sources, and may be natural or synthetic. The translation initiation region can be derived from the transcription initiation region or from a structural gene. To facilitate the identification and screening of transgenic plant cells or plants, the plant expression vector can be modified. For example, genes that can express in plants and encode enzyme that can produce color changes or luminescent compounds in plants (such as the *GUS* gene and luciferase gene), antibiotic marker genes (like *nptII* for kanamycin and related antibiotics resistance, *bar* for glufosinate herbicide resistance, *hph* for hygromycin resistance, *dhfr* for methotrexate resistance, and EPSPS for glyphosate resistance), or chemically resistant marker genes (such as herbicide resistance genes), genes for invertase, which provide the ability to metabolize mannose, can be added. Additionally, the, can be included Considering the safety of transgenic plants, the transformed plants can be directly screened under adverse conditions without adding any selective marker genes.

The microorganism may be yeast, bacteria, algae or fungi, such as *A*. *tumefaciens.* The recombinant microorganism may be a microorganism containing the above-mentioned expression cassette or the above-mentioned recombinant vector.

In the above use, the regulation of plant disease resistance is to improve plant disease resistance; the improvement of plant disease resistance is reflected in: the higher the RCR1 protein content and/or activity in the plant or the higher the *RCR1* gene expression, the greater the resistance of the plant; further reflected in: the higher the RCR1 protein content and/or activity in the plant or the higher the *RCR1* gene expression, the higher the plant's resistance to *P. brassicae.*

In a sixth aspect, the present invention provides a new use of the substance represented by Q1) or Q2):
Q1) Substance that inhibits or reduces the activity and/or content of RCR1 protein in a plant;
Q2) Substance that inhibits the expression of the RCR1 protein-encoding gene in a plant or knocks out the RCR1 protein-encoding gene in a plant.

The present invention provides use of the substance represented by Q1) or Q2) in reducing plant disease resistance or cultivating a transgenic plant or gene-edited plant with reduced disease resistance.

In the above use, the substance that inhibits or reduces the activity and/or content of RCR1 protein in a plant can be any substance that can cause the loss of RCR1 protein activity in plants, such as proteins, polypeptides or small molecule compounds (such as protein activity inhibitors) that inhibit RCR1 protein synthesis or promote RCR1 protein degradation or inhibit RCR1 protein function.

The substance that inhibits the expression of the RCR1 protein-encoding gene can be any substance that can prevent the expression of the RCR1 protein-encoding gene in plants, such as substances that silence the RCR1 protein-encoding gene in plants (such as miRNA, siRNA, dsRNA, shRNA, etc.).

The substance that knocks out the RCR1 protein-encoding gene can be any material that achieves the functional protein product of the *RCR1* gene not being produced by the host cell. Specific methods include removing all or part of the coding gene sequence, introducing frame shift mutations to prevent the production of functional proteins, removing or altering regulatory components (such as promoter editing) so that the coding gene sequence is not transcribed, and preventing translation by binding to mRNA. Typically, knock outs are performed at the level of genomic DNA, so that future generations of cells also permanently carry the knock out.

Further, the substance for knocking out the RCR1 protein-encoding gene can be any substance that can mutate the RCR1 protein-encoding gene in plants (the mutation form can be deletion mutation and/or insertion mutation and/or base substitution), thereby losing activity, such as zinc finger protein ZFN gene editing system or TALENs gene editing system or CRISPR/Cas9 gene editing system, etc.

Furthermore, the material that knocks out the RCR1 protein-encoding gene is a CRISPR/Cas9 gene editing vector that knocks out the RCR1 protein-encoding gene in plants.

In a specific embodiment of the present invention, the CRISPR/Cas9 gene editing vector for knocking out the RCR1 protein-encoding gene in plants is a recombinant vector pHEE401E-RCR1, and the recombinant vector pHEE401E-RCR1 is a vector obtained by inserting the DNA molecule shown in Sequence 4 at the BsaI restriction site of the pHEE401E vector.

In the above use, the reduction of plant disease resistance is reflected in: disease-resistant plants with RCR1 protein deletion or *RCR1* gene knock out showing disease susceptibility; further reflected in clubroot-resistant plants with RCR1 protein deletion or *RCR1* gene knock out showing infecting clubroot disease.

In a seventh aspect, the present invention provides a method for cultivating a transgenic plant or gene-edited plant with improved disease resistance.

The method for cultivating a transgenic plant or gene-edited plant with improved disease resistance provided by the present invention comprising the steps of increasing the content and/or activity of RCR1 protein in a recipient plant to obtain a transgenic plant or gene-edited plant; the transgenic plant or gene-edited plant; the disease resistance of the transgenic plant or gene-edited plant being higher than that of the recipient plant.

Further, the disease resistance of the transgenic plant or gene-edited plant is higher than that of the recipient plant, as reflected in any one of the following X1)-X2):
X1) an incidence rate of the transgenic plant or gene-edited plant lower than that of the recipient plant;
X2) a disease index of the transgenic plant or gene-edited plant lower than that of the recipient plant.

Furthermore, the method for increasing the content and/or activity of RCR1 protein in a recipient plant is to overexpress RCR1 protein in a recipient plant.

The overexpression method may be to introduce an encoding gene of RCR1 protein into the recipient plant.

Furthermore, the encoding gene of RCR1 protein is introduced into the recipient plant through a recombinant vector.

The recombinant vector comprises a promoter for initiating expression of the encoding gene of RCR1 protein.

The encoding gene of RCR1 protein is shown in Sequence 3 at positions 3644-4090.

The nucleotide sequence of the promoter is shown in positions 1-3643 of Sequence 3, or positions 1-6131 of Sequence 5.

In a specific embodiment of the present invention, the encoding gene of RCR1 protein is introduced into the recipient plant through the recombinant vector pCAMBIA1300-p3.6k*RCR1*. The recombinant vector pCAMBIA1300-p3.6k*RCR1* is a vector obtained after inserting the DNA molecule shown in Sequence 3 into the KpnI restriction site of the pCAMBIA1300 vector.

In another specific embodiment of the present invention, the RCR1 protein-encoding gene is introduced into the recipient plant through the recombinant vector pCAMBIA1300-p6k*RCR1*. The recombinant vector pCAMBIA1300-p6k*RCR1* is a vector obtained after inserting the DNA molecule shown in Sequence 5 into the KpnI restriction site of the pCAMBIA1300 vector.

In an eighth aspect, the present invention provides a method for cultivating a transgenic plant or gene-edited plant with reduced disease resistance.

The method for cultivating a transgenic plant or gene-edited plant with reduced disease resistance provided by the present invention comprises the steps of reducing the activity and/or content of RCR1 protein in a recipient plant to obtain a transgenic plant or gene-edited plant; the transgenic plant or gene-edited plant having disease resistance lower than that of the recipient plant.

Further, the disease resistance of the transgenic plant or gene-edited plant is lower than that of the recipient plant, as shown in any one of the following Y1)-Y2):
Y1) an incidence rate of the transgenic plant or gene-edited plants higher than that of the recipient plant;
Y2) a disease index of the transgenic plant or gene-edited plant higher than that of the recipient plant.

Furthermore, the method for reducing the activity and/or content of RCR1 protein in the recipient plant is achieved by introducing the substance that inhibits or reduces the activity and/or content of RCR1 protein in the plant, or the substance that inhibits the expression of the RCR1 protein encoding gene in the plant, or the substance that knocks out the RCR1 protein encoding gene in the plant, into the recipient plant.

Furthermore, the substance that knocks out the RCR1 protein-encoding gene is a CRISPR/Cas9 gene editing vector that knocks out the RCR1 protein-encoding gene in plants.

In a specific embodiment of the present invention, the CRISPR/Cas9 gene editing vector for knocking out the RCR1 protein-encoding gene in plants is a recombinant vector pHEE401E-RCR1, and the recombinant vector pHEE401E-*RCR1* is a vector obtained by inserting the DNA molecule shown in Sequence 4 at the BsaI restriction site of the pHEE401E vector..

Transgenic plants or gene-edited plants prepared by any of the above methods also belong to protection scope of the present invention.

In any of the above uses or methods, the plant is a monocotyledonous plant or a dicotyledonous plant, and the dicotyledonous plant can be a cruciferous plant; the cruciferous plant can specifically be *A*. *thaliana, B. napus,* or cabbage. In specific embodiments of the present invention, the *A*. *thaliana* can be Colombian ecotype Col-0 or Est-1; the *B. napus* can be Zhongshuang 11 (ZS11).

In any of the above-mentioned applications or methods, the disease resistance is resistance to clubroot fungus; the clubroot fungus is specifically the physiological race No. 4 of clubroot fungus.

### BRIEF DESCRIPTION OF THE FIG.S

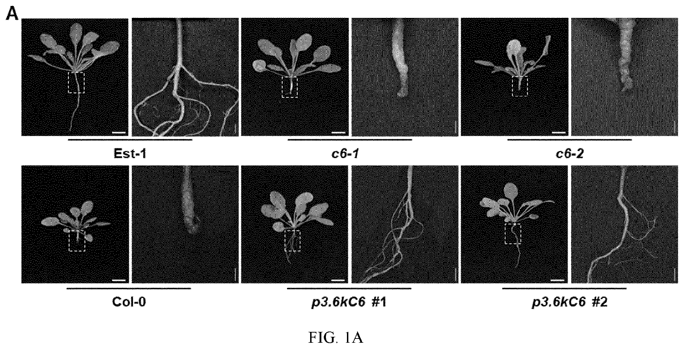

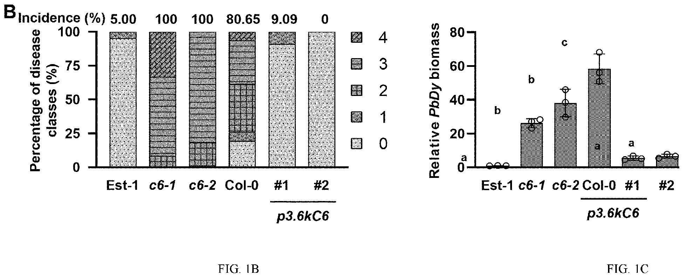

FIG. 1 shows the resistance analysis of transgenic *A*. *thaliana* plants to clubroot disease. **A** shows Est-1, *RCR1*-knock out plants *rcr1-1* and *rcr1-2,* Col-0, and *RCR1* overexpression plants *p3.6kRCR1_Col-0* #1 and *p3.6kRCR1_*Co1-0 #2 were inoculated with *P. brassicae PbDy,* a strain collected from Dayi, Sichuan. The symptoms of the inoculated plants were observed and photographed 21 days post inoculation. Wherein, white line: 1 cm; red line, 1 mm. **B and C** shows incidence rate and disease index (B) and relative biomass (C) (n≥12) of Est-1, RCR1 knock out plants *rcr1-1* and *rcr1-2,* Col-0, and *RCR1* overexpression plants *p3.6kRCR1*_Col-0#1 and *p3.6kRCR1*_Col-0#2 21 days after inoculation with *P. brassicae.*

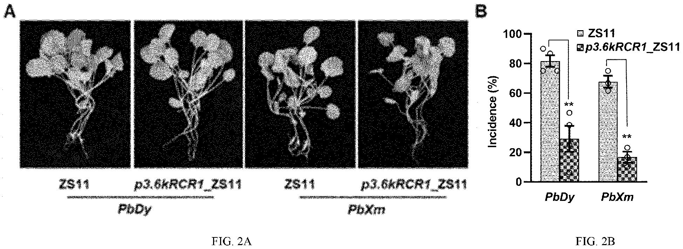

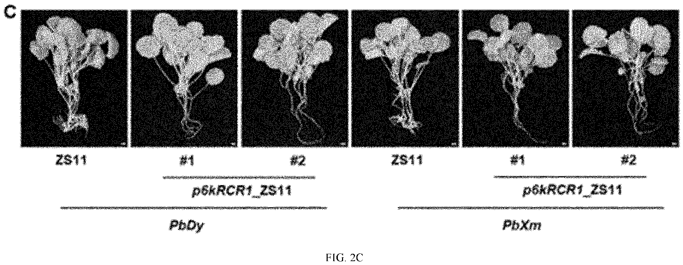

FIG. 2 shows the resistance analysis of transgenic *B. napus* plants to *P. brassicae.* **A** shows ZS11 and *p3.6kRCR1*_ZS11 were inoculated with *PbDy,* the strain collected from Dayi region, Sichuan and *PbXm,* the strain collected from Xinmin region, Shenyang. The symptoms of the indicated inoculated plants were observed and photographed 21 days post inoculation. *p3.6kRCR1*_ZS11 is a transgenic plant expressing *p3.6kRCR1* in the Zhongshuang11(ZS11)background. **B** shows the incidence rate of ZS11 and *p3.6kRCR1*_ZS11 21 days after inoculation with *P. brassicae* (n≥12). **C** shows ZS11 and *p6kRCR1*_ZS11 were inoculated with *PbDy* the strain collected from Dayi region, Sichuan and *PbXm,* the strain collected from Xinmin region, Shenyang. The symptoms of the indicated inoculated plants were observed and photographed 21 days post inoculation. *p6kRCR1_*ZS11#1 and *p6kRCR1*_ZS11#2 are transgenic plants expressing *p6kRCR1* under the ZS11 background.

### PREFERRED EMOBIDMENTS OF THE INVENTION

The following examples facilitate a better understanding of the present invention, but do not limit the present invention. The test methods in the following examples are all conventional methods unless otherwise specified. The test materials used in the following examples were all purchased from conventional biochemical reagent stores unless otherwise specified. The quantitative experiments in the following examples were repeated three times, and the results were averaged.

The pCAMBIA1300 vector in the following examples is described in the document *"*Liang, XX. et al. Ligand-triggered de-repression of Arabidopsis heterotrimeric G proteins coupled to immune receptor kinases. Cell Research 28, 529-543 (2019)." This biological material can be available from the Institute of Genetics and Developmental Biology of the Chinese Academy of Sciences. This biological material is only used to repeat the experiments related to the invention and cannot be used for other purposes.

The pCBC-DT1T2 vector and pHEE401E vector in the following examples are both described in the document "Editor, D. CRISPR-GE: aconvenient software toolkit for CRISPR-based genome editing. Mol. Plant 10, 1246-1249 (2017)". This biological material can be available from the Institute of Genetics and Developmental Biology of the Chinese Academy of Sciences. This biological material is only used to repeat the relevant experiments related to the invention and cannot be used for other purposes.

The *A*. *tumefaciens* GV3101 in the following examples is described in the document *"*Liang, XX. et al. Ligand-triggered de-repression of Arabidopsis heterotrimeric G proteins coupled to immune receptor kinases. Cell Research 28, 529-543 (2019)". This biological material can be available from the Institute of Genetics and Developmental Biology of the Chinese Academy of Sciences. This biological material is only used to repeat the relevant experiments related to the invention and cannot be used for other purposes.

The *B. napus* variety Zhongshuang 11 (ZS11) in the following examples is described in the document *"*Sun, FM. et al. The high-quality genome of Brassica napus cultivar'ZS11' reveals the introgression history in semi-winter morphotype. Plant J. 92, 452-468(2017)". This biological material can be available from the Institute of Genetics and Developmental Biology of the Chinese Academy of Sciences. This biological material is only used to repeat the relevant experiments related to the invention and cannot be used for other purposes.

The *P. brassicae PbXm* in the following examples is described in the document *"*Shah, N., Sun, J., Yu, S. et al. Genetic variation analysis of field isolates of clubroot and their responses to Brassica napus lines containing resistant genes CRb and PbBa8.1 and their combination in homozygous and heterozygous state. Mol Breeding 39, 153(2019)". This biological material can be available from the Institute of Genetics and Developmental Biology, Chinese Academy of Sciences. This biological material is only used to repeat the relevant experiments related to the invention and cannot be used for other purposes.

The amino acid sequence of the RCR1 protein in the following examples is Sequence 2, the nucleotide sequence of its encoding gene corresponds to positions 1-447 in Sequence 1, and its genome sequence corresponds to positions 3644-4090 in Sequence 3.

Example 1. Obtaining *RCR1*-knock out *A*. *thaliana* and *RCR1*-overexpressing *A*. *thaliana* and analysis of their resistance to *P. brassicae*

One. Obtaining *RCR1* -knock out *A*. *thaliana* and *RCR1* -overexpression *A*. *thaliana*

### 1. Construction of RCR1 -knock out vector

### 1) Obtaining the knock out vector target

The website http://skl.scau.edu.cn/ was used to design targets for knocking out the *RCR1* gene. The target sequences are as follows:
Target 1: 5'-TGGAGTAACCCTAGAATTCCGG-3' (Sequence 6);
Target 2: 5'-GTTTAGTCCCAAGGCTCATTGG-3' (Sequence 7).

### 2) Construction of vector

Primers were designed according to the designed target. The primer sequences are as follows:
Targte1-BsF:
   ATATATGGTCTCGATTGTGGAGTAACCCTAGAATTCGTT (Sequence 8);
Targte1-F0:
   TGTGGAGTAACCCTAGAATTCGTTTTAGAGCTAGAAATAGC (Sequence 9);
Targte2-R0:
   AACATGAGCCTTGGGACTAAACCAATCTCTTAGTCGACTCTAC (Sequence 10);
Targte2-BsR: ATTATTGGTCTCGAAACATGAGCCTTGGGACTAAACC (Sequence 11).

Four-primer PCR amplification was performed using the pCBC-DT1T2 vector as a template, and the target fragment was recovered using agarose gel electrophoresis, and then the recovered target fragment was inserted into the pHEE401E vector using a restriction-ligation cloning approach, generating a recombinant vector pHEE401E-*RCR1*, that is the *RCR1*-knock out vector. It was then sequenced.

The system for restriction-ligation cloning was as follows: target fragment 2 µl, pHEE401E vector 2 µl, 10×T4 ligase buffer1.5 µl, 10×BSA1.5 µl, BsaI1 µl, T4 ligase 1µl, and sterile water 6 µl.

The sequencing results show that the recombinant vector pHEE401E-*RCR1* was a vector obtained by inserting the DNA molecule shown in Sequence 4 into the BsaI restriction site of the pHEE401E vector.

### 2. Construction of RCR1 gene expression vector

### 1) Obtaining RCR1 gene fragment

The DNA of the ecotype Est-1 of *A*. *thaliana* was extracted and amplified by PCR using primers RCR1-F-3.6k and RCR1-R. A PCR product of 4800 bp in size was obtained, and its nucleotide sequence is shown in Sequence 3. This sequence, designated as the *p3.6kRCR1* gene, encompasses a *RCR1* promoter region, a sequence of approximately 3.6 kb, a *RCR1* coding region, a sequence of approximately 1.1 kb and a UTR region. The primer sequences are as follows:
RCR1-F-3.6k:
   5'-CGAATTCCGAATTCGAGCTCGATCTGGTAATGCGCTGATTCTATCG-3' (Sequence 12);
RCR1-R:
   5'-TTCATTTCGAAGGTACCGGAAACTAGGTGTTGGGATCTGGG-3' (Sequence 13).

### 2) Obtaining RCR1 gene expression vector

The pCAMBIA1300 vector was digested with KpnI single enzyme and recovered to obtain the pCAMBIA1300 vector backbone; using in-Funion homologous recombination technology, the pCAMBIA1300 vector backbone was reacted with the PCR product of 4800bp in size. The expression vector pCAMBIA1300-*p3.6kRCR1* was obtained and sequenced.

The sequencing results show that the recombinant vector pCAMBIA1300-p3.6k*RCR1* is a vector obtained by inserting the DNA molecule shown in Sequence 3 into the KpnI restriction site of the pCAMBIA1300 vector. In the DNA molecule shown in Sequence 3, positions 1-3643 correspond to the promoter sequence, positions 3644-4090 correspond to the *RCR1* genome sequence, and positions 4091-4800 correspond to the 3'UTR region.

### 3. Obtaining transgenic A. thaliana

### 1) Obtaining A. tumefaciens carrying pHEE401E-RCR1 or pCAMBIA1300-p3.6kRCR1.

The method of electroporation transformation was used to introduce the recombinant vector pHEE401E-*RCR1* obtained in step 1 and the pCAMBIA1300-*p3.6kRCR1* obtained in step 2 into *A*. *tumefaciens* GV3101. The transformed strain was smeared on an LB solid culture dish containing kanamycin and gentamicin, cultured at 28°C for 36 hours. Positive monoclonal strains were screened to obtain *A*. *tumefaciens* GV3101 carrying pHEE401E-*RCR1* and pCAMBIA1300-*p3.6kRCR1* of *A*. tumefaciens GV3101, respectively.

### 2) Obtaining transgenic A. thaliana

Using the *A*. *thaliana* transformation method mediated by *A*. *tumefaciens,* the *A*. *tumefaciens* GV3101 carrying pHEE401E-*RCR1* obtained in step 1) was transformed into *A*. *thaliana* ecotype Est-1 (clubroot-resistant material), and the *A*. *tumefaciens* carrying pCAMBIA1300-*p3.6kRCR1* obtained in step 1) was transformed into *A*. *thaliana* ecotype Col-0 (clubroot-susceptible material). The specific steps were as follows:
I. Preparation of *A*. *thaliana* transformation receptors: *A*. *thaliana* in the flowering stage were selected, the flowers that have opened were picked, and the remaining *A*. *thaliana* buds were used as receptors for *A*. *tumefaciens* infection.
II. Cultivation of *A*. *tumefaciens:* A small amount of bacterial solution from the *A*. *tumefaciens* storage solution was taken and cultured in streaks on LB solid medium (containing 50 mg/l kanamycin and 50 mg/l gentamicin) at 28°C in the dark for 12 hours. A few bacterial cells were picked and inoculated into 2 ml of LB liquid culture medium (containing the corresponding antibiotics) and cultured at 28°C for 12 hours. Then 2 ml of LB liquid culture medium was transferred to 300 ml of LB liquid culture medium (containing the corresponding antibiotics) and cultured for about 10 hours, centrifuged at 4,000rpm to collect the bacterial solution. The bacterial solution was diluted with 5% sucrose solution to an OD of about 0.9, and a surfactant (silwet L-77) was added at a ratio of 17/10000 to prepare for transformation.
III. *A*. *tumefaciens* infection of *A*. *thaliana* flower buds: The diluted bacterial solution was put into a cylindrical glass bottle. The *A*. *thaliana* containing only flower buds was placed upside down in the bacterial solution for 4-5 minutes, and then laid horizontally for 24 hours in the dark. After 24 hours of protection from light, the plant grew vertically.
IV. Screening of resistant plants: The seeds received from the transgenic plants were spread evenly on a 1/2 MS medium containing 25mg/L hygromycin B and 50mg/L carbenicillin for screening, and after 10 days, normal growth seedlings were selected to move to the greenhouse for cultivation.

### 3) Obtaining RCR1-knock out A. thaliana

Transgenic *A*. *thaliana* that has grown for about two weeks were selected, and then 1-2 leaves were picked to extract total DNA using the CTAB method, and the DNA fragments in the genome were amplified by using primer RCR1-de-F and primer RCR1-de-R . It was sequenced to determine the mutation site, and finally two different mutant forms of *RCR1*-knock out *A*. *thaliana* lines were obtained, named *rcr1-1* and *rcr1-2* respectively. The primer sequences are as follows:
RCR1-de-F: 5'-ATGGAGACTGTCTCCGCCGT-3' (Sequence 14);
RCR1-de-R: 5'-TTAAACTGGTGGTAACTGA-3' (Sequence 15).

*RCR1* -knock out *A*. *thaliana rcr1-1* is an *A*. *thaliana* mutant with a homozygous mutation in the RCR1 gene (the same mutation occurred in both chromosomes). The only difference in the genome sequence from the wild-type *A*. *thaliana* ecotype Est-1 was that a base deletion of 145 bp occurred at positions 116-260 of the gene encoding RCR1 protein (Sequence 1).

*RCR1*-knock out *A*. *thaliana rcr1-2* is an *A*. *thaliana* mutant with a homozygous mutation in the *RCR1* gene (the same mutation occurred in both chromosomes). The only difference in the genome sequence from the wild-type *Arabidopsis* ecotype Est-1 was that a base mutation occurred at position 260 of the gene encoding RCR1 protein (Sequence 1), and the base T was mutated into base A at this position.

### 4) Obtaining RCR1-overexpression in A. thaliana

Transgenic *A*. *thaliana* that has grown for about two weeks was selected, and then 1-2 leaves were picked to extract total DNA using the CTAB method, and then PCR was performed to amplify the DNA in the genome to detect the integration of the target gene into the genome. The primer pair used for PCR amplification was RCR1-F and 1300-R. The amplification product was the connection fragment located at the 3' end of *RCR1* and the pCAMBIA1300 vector. The amplified transgenic *A*. *thaliana* with a size of approximately 1000 bp was *p3.6kRCR1* overexpression *A*. *thaliana,* termed as *p3.6kRCR1*_Col-0. Two independent *p3.6kRCR1* overexpression *A*. *thaliana* lines were termed *p3.6kRCR1_*Col-0#1 and *p3.6kRCR1_*Col-0#2, respectively. T₂ generation was used as the experimental materials for the following clubroot resistance analysis. The primer sequences are as follows:
RCR1-F: 5'-ATGGAGACTGTCTCCGCCG-3' (Sequence 16);
1300-R: 5'-GTAGGATTCTGGTGTGTGCGC-3' (Sequence 17).

### Two. Analysis of resistance of RCR1-knock out A. thaliana and RCR1-overexpression A. thaliana to P. brassicae

### 1. P. brassicae inoculation

Est-1, Col-0, and *RCR1*-knock out *A*. *thaliana rcr1-1, rcr1-2, RCR1 overexpress A*. *thaliana p3.6kRCR1_Col-0* #1 and *p3.6kRCR1*_Col-0 #2 obtained in step one, were used as experimental materials for *P. brassicae* inoculation experiment respectively. Before inoculation, each experimental material was cultured under conventional conditions (12 hours of light, 12 hours of darkness, at a temperature of 22°C). When the plants had grown for about 14 days, *P. brassicae* infection experiment was conducted. The specific steps are as follows:
1) The roots pieces of the collected plants containing *P. brassicae* (recorded as *P. brassicae PbDy,* identified as physiological race No. 4) from the Dayi region of Sichuan province were cut into small pieces with a blade, added with water, and then broken in a tissue crusher .
2) The mixture containing bacterial solution and broken tissue was filtered by using 8 layers of gauzer to obtain relatively clean *P. brassicae PbDy* solution.
3) The spore concentration of the *P. brassicae PbDy* in the bacterial solution was measured by using a hemocytometer.
4) The concentration of *P. brassicae PbDy* was diluted to 1.0×10⁷ /mL, and then the root-drenching method was used to inoculate *A*. *thaliana,* that is: 1 mL of the diluted bacterial solution was absorbed by using a pipette, and poured on the roots of *A*. *thaliana.*

### 2. Disease index and incidence statistics

The incidence rate and disease index were calculated by using the grading system of *A*. *thaliana P. brassicae.* The statistical standards of the grading system of *A*. *thaliana-P. brassicae* are as follows: Grade 0, the roots of *A*. *thaliana are* not infected and the structure of root system is intact; Grade 1, there are small nodules on the lateral roots and the main root is not affected; Grade 2, there are lumps on the lateral roots and main roots , but some root structures are intact and unaffected; Grade 3, both the main root and lateral roots are infected; Grade 4, the lateral roots and root hairs of *A*. *thaliana* are completely damaged, leaving only an enlarged main root in rod shape. The calculation formula of disease incidence = (number of diseased plants/total number of inoculated plants) × 100%; the calculation formula of disease index = (Σ (number of disease grade × number of diseased plants at this grade))/(total number of inoculated plants × number of the highest grade 4)×100.

### 3. Analysis of relative biomass of P. brassicae

After inoculation with *PbDy* for 21 days, the underground parts of each material were collected, the total DNA of the underground parts was extracted using the CTAB method, and then quantitative real-time PCR was used to detect the expression of *A*. *thaliana* housekeeping gene *At-Actin* and *P. brassicae* housekeeping gene *Pb-Actin* in different materials. The primer sequences are as follows:
Actin-RT-F: 5'-AACTCTCCCGCTATGTATGTCG-3' (Sequence 18);
Actin-RT-R: 5'-AACCCTCGTAGATTGGCACA-3'(Sequence 19);
Pb-Actin-RT-F: 5'-CACCGACTACCTGATGAA-3' (Sequence 20);
Pb-Actin-RT-R: 5'-CAGCTTCTCCTTGATGT-3' (Sequence 21).

The disease symptoms, incidence rate and disease index of the plants 21 days after the inoculation of the *P. brassicae,* and the relative biomass of the *P. brassicae* are shown in FIG. 1A-C. The results show that: 21 days after the inoculation, the transgenic material *p3.6kRCR1_Col* -0#1 and #2 heterologously expressing *RCR1* showed similar phenotypes to the resistant material Est-1, that is, root development was basically normal, and only a small number of plants showed mild symptoms; while in the background of the resistant material Est-1, the two *RCR1* gene knock out mutants *rcr1-1* and *rcr1-2* showed a similar phenotype to the clubroot susceptible material Col-0, that is, spindle-shaped tumors formed in the roots, and almost all plants showed severe symptoms. At the same time, the relative biomass of Rhizobium in each material was also consistent with its phenotype (FIG. 1C). This result indicates that *RCR1* mediates *A*. *thaliana* resistance to clubroot.

### Example 2. Obtaining RCR1-overexpressing B. napus and analysis of its resistance to P. brassicae

### one . Obtaining RCR1 overexpressing B. napus

### 1. Construction of RCR1 gene expression vector

### 1) Obtaining long promoter RCR1 gene fragment

The DNA of *A*. *thaliana* ecotype *Est-1* was extracted and performed PCR amplification using the primer RCR1-F-6k and the primer RCR1-R in Example 1 to obtain a PCR product of 7288 bp in size, and its nucleotide sequence is shown in Sequence 5, termed p6k*RCR1* gene, including a promoter sequence of approximately 6 kb, an encoding region of approximately 1.1 kb, and a UTR region. The primer sequences are as follows:
RCR1-F-6k:
5'-CGAATTCCGAATTCGAGCTCCCTAATCCGATCGAGTACAA-3' (Sequence 22).

### 2) Obtaining RCR1 gene expression vector

,A recombinant vector pCAMBIA1300-p6k*RCR1* was obtained by recombining *p6kRCR1* into the pCAMBIA1300 vector backbone according to the method in step 2 of step one of Example 1, and then was sequenced.

The sequencing results show that the recombinant vector pCAMBIA1300-p6k*RCR1* is a vector obtained by inserting the DNA molecule shown in Sequence 5 into the KpnI restriction enzyme cutting site of the pCAMBIA1300 vector. In the DNA molecule shown in Sequence 5, positions 1-6131 correspond to the promoter sequence, positions 6132-6578 correspond to the *RCR1* genome sequence, and positions 6578-7288 correspond to the 3'UTR region.

### 2. Obtaining RCR1 overexpressing B. napus

According to the method in step 3 of step one of Example 1, the recombinant vector pCAMBIA1300-p3.6k*RCR1* was introduced into the clubroot-susceptible material Zhongshuang 11 (ZS11). The transgenic experiment was entrusted to Wuhan Boyuan Biotechnology Co., Ltd to complete. After obtaining the transgenic seedlings, the transgenic seedlings were screened according to the method of Example 1 to obtain the transgenic positive seedlings, and *RCR1* overexpressing *B. napus p3.6kRCR1_ZS11* was obtained.

According to the method in step 3 of step one of Example 1, the recombinant vector pCAMBIA1300-p6k*RCR1* was introduced into clubroot-susceptible material Zhongshuang 11 (ZS11). The transgenic experiment was entrusted to Wuhan Boyuan Biotechnology Co., Ltd to complete.. After obtaining the transgenic seedlings, the transgenic seedlings were screened according to the method of Example 1 to obtain *RCR1*-overexpressing *B. napusp6kRCR1*_ZS11#1 and p6k*RCR1*_ZS11#2.

### Two. Analysis of resistance of RCR1-overexpressing B. napus to P. brassicae

According to the method in step two of Example 1, *B. napus p3.6kRCR1_ZS11, p6kRCR1*_ZS11 and ZS11 were overexpressed with *RCR1* driven by promoters of different lengths and inoculated with *P. brassicae PbDy* or *PbXm,* where *PbDy* was *P. brassicae* from Dayi area, Sichuan and *PbXm* was from Xinmin area, Shenyang(identified as physiological race No. 4).

The plant phenotype was observed 21 days after inoculation, and it was found that ZS11 showed obvious susceptibility to two kinds of *P. brassicae, PbDy* and *PbXm,* that is, spindle-shaped tumors were formed in the roots of the plants, while *RCR1* overexpressed *B. napus* p3.6k*RCR1*_ZS11 and *p6kRCR1*_ZS11 which were driven by promoters of different lengths were inoculated with the two bacteria, their underground parts developed normally (FIG. 2A). And when inoculated with *P. brassicae* from different regions, the incidence of *RCR1* overexpression *B. napus* p3.6*RCR1*_ZS11 driven by the 3.6k promoter was significantly lower than that of ZS11 (FIG. 2B). It shows that the clubroot disease resistance gene *RCR1* can be used to improve the *B. napus* clubroot disease resistance. It further illustrates that the *RCR1* gene driven by the p3.6k*RCR1* promoter is a key element in plant clubroot resistance, and its expression in other clubroot-susceptible plants can improve or restore plant clubroot resistance.

The above results show that the *RCR1* gene and its promoter p3.6k*RCR1* are related to plant clubroot resistance. The *RCR1* gene and the *RCR1* gene driven by the p3.6k*RCR1* promoter can be used for the cultivation and identification of disease-resistant plant varieties in agricultural production, playing an important role in the field of plant disease-resistance breeding.

### INDUSTRIAL APPLICATION

The present invention demonstrates through experiments that when RCR1-transgenic *A*. *thaliana* or RCR1-transgenic *B. napus* is infected with *P. brassicae,* the resistance to *P. brassicae* is significantly enhanced as compared with wild-type *A*. *thaliana* or *B. napus*; and the resistance to *P. brassicae* of *RCR1* gene-knock out *A*. *thaliana* is significantly reduced compared with the wild-type *A*. *thaliana,.* It is indicated that the RCR1 gene is related to the resistance of the plant to *P. brassicae,* and the RCR1 protein and the coding gene thereof can be used for cultivating clubroot-resistant varieties in agricultual production. The present invention has great significance for cultivating disease-resistant varieties, especially for breeding new varieties of clubroot-resistant Brassicaceae crops, and can be used for cultivating and identifying disease-resistant plant varieties required by agricultual production.

## Claims

1. A DNA molecule comprising, in order from the 5' end, a transcription regulatory element of *RCR1* gene and a *RCR1* gene; wherein the transcription regulatory element of *RCR1* gene is the following A1) or A2):
A1) a DNA molecule as shown at positions 1-3643 of Sequence 3;
A2) a DNA molecule having 50% or higher identity to the nucleotide sequence defined in A1) and having transcriptional regulatory function;
and the *RCR1* gene is the following B1) or B2):
B1) a DNA molecule as shown at positions 3644-4090 of Sequence 3;
B2) a DNA molecule having 50% or higher identity to the nucleotide sequence defined in B1) and having the same function as the nucleotide sequence defined in B1).

2. The DNA molecule according to claim 1, wherein: the DNA molecule comprises in order from the 5' end, the transcription regulatory element of *RCR1* gene, the *RCR1* gene, and a 3'UTR region;
wherein the nucleotide sequence of the 3'UTR region is the following C1) or C2):
C1) a DNA molecule as shown at positions 4091-4800 of Sequence 3;
C2) a DNA molecule having 50% or higher identity to the nucleotide sequence defined in C1) and having the same function as the nucleotide sequence defined in C1).

3. The DNA molecule according to claim 1 or 2, wherein: the nucleotide sequence of the DNA molecule is the following D1) or D2):
D1) a DNA molecule as shown in Sequence 3;
D2) a DNA molecule having 50% or higher identity to the nucleotide sequence defined in D1) and having the same function as the nucleotide sequence defined in D1).

4. A biological material related to the DNA molecule according to any one of claims 1-3, wherein the biological material is a recombinant vector comprising the DNA molecule of any one of claims 1-3, a recombinant microorganism comprising the DNA molecule of any one of claims 1-3, or a transgenic plant cell line comprising the DNA molecule of any one of claims 1-3.

5. Use of the DNA molecule according to any one of claims 1-3 or the biological material according to claim 4 in the following M1) or M2):
M1) regulating plant disease resistance;
M2) cultivating a transgenic plant or gene-edited plant with enhanced disease resistance.

6. Use of a RCR1 protein or a biological material related to RCR1 protein in the following M1) or M2):
M1) regulating plant disease resistance;
M2) cultivating a transgenic plant or gene-edited plant with enhanced disease resistance;
wherein the biological material is a nucleic acid molecule encoding RCR1 protein, or an expression cassette comprising the nucleic acid molecule, a recombinant vector comprising the nucleic acid molecule, a recombinant microorganism comprising the nucleic acid molecule, or a transgenic plant cell line comprising the nucleic acid molecule;
and the RCR1 protein is as defined in N1) or N2) or N3) or N4):
N1) a protein having an amino acid sequence of Sequence 2;
N2) a fusion protein obtained by attaching a tag to the N-terminus and/or C-terminus of the protein of Sequence 2;
N3) a plant disease resistance-related protein derived from the amino acid sequence of Sequence 2 by substitution, and/or deletion, and/or addition of one or more amino acid residues;
N4) a plant disease resistance-related protein having 75% identity to Sequence 2.

7. The use according to claim 6, wherein: the nucleic acid molecule encoding the RCR1 protein is a DNA molecule as shown in the following P1) or P2) or P3):
P1) a DNA molecule as shown in Sequence 1;
P2) a DNA molecule as shown at positions 3644-4090 of Sequence 3;
P3) a DNA molecule having 50% or higher identity to the nucleotide sequence defined in P1) or P2) and encoding the RCR1 protein.

8. Use of a substance as shown in Q1) or Q2) in reducing plant disease resistance or cultivating a transgenic plant or gene-edited plant with reduced disease resistance:
Q1) a substance that inhibits or reduces the activity and/or content of a RCR1 protein in a plant;
Q2) a substance that suppresses the expression of a RCR1 protein-encoding gene in a plant or knocks out the RCR1 protein-encoding gene in a plant;
wherein the RCR1 protein is as defined in N1) or N2) or N3) or N4):
N1) a protein having an amino acid sequence of Sequence 2;
N2) a fusion protein obtained by attaching a tag to the N-terminus and/or C-terminus of the protein of Sequence 2;
N3) a plant disease resistance-related protein derived from the amino acid sequence of Sequence 2 by substitution, and/or deletion, and/or addition of one or more amino acid residues;
N4) a plant disease resistance-related protein having 75% identity to Sequence 2.

9. The use according to any one of claims 5-8, wherein: the disease resistance is resistance to *Plasmodiophora brassicae.*

10. The use according to any one of claims 5-9, wherein: the plant is as defined in M1) or M2) or M3) or M4):
M1) a monocotyledonous or dicotyledonous plant;
M2) a Brassicaceae plant;
M3) an *Arabidopsis thaliana*;
M4) a *Brassica napus.*

11. A method for cultivating a transgenic plant or gene-edited plant with enhanced disease resistance, comprising the following steps:
increasing the content and/or activity of the RCR1 protein in a recipient plant to obtain a transgenic or gene-edited plant, wherein the disease resistance of the transgenic or gene-edited plant is higher than that of the recipient plant;
wherein the RCR1 protein is as defined in N1) or N2) or N3) or N4):
N1) a protein having an amino acid sequence of Sequence 2;
N2) a fusion protein obtained by attaching a tag to the N-terminus and/or C-terminus of the protein of Sequence 2;
N3) a plant disease resistance-related protein derived from the amino acid sequence of Sequence 2 by substitution, and/or deletion, and/or addition of one or more amino acid residues;
N4) a plant disease resistance-related protein having 75% sequence identity to Sequence 2.

12. The method according to claim 11, wherein: the transgenic plant or gene-edited plant having a higher disease resistance than the recipient plant reflects in any one of the following X1) or X2):
X1) a lower disease incidence in the transgenic or gene-edited plant compared to the recipient plant;
X2) a lower disease index in the transgenic or gene-edited plant compared to the recipient plant.

13. The method according to claim 11 or 12, wherein: the method for increasing the content and/or activity of RCR1 protein in the recipient plant is to overexpress the RCR1 protein in the recipient plant.

14. The method according to any one of claims 11-13, wherein: the disease resistance is resistance to *Plasmodiophora brassicae.*

15. The method according to any one of claims 11-14, wherein: the plant is as defined in M1) or M2) or M3) or M4):
M1) a monocotyledonous or dicotyledonous plant;
M2) a Brassicaceae plant;
M3) an *Arabidopsis thaliana;*
M4) a *Brassica napus.*

16. A transgenic plant or gene-edited plant prepared by the method according to any one of claims 11-15.

17. A method for cultivating transgenic plant or gene-edited plant with reduced disease resistance, comprising the steps of: reducing the activity and/or content of a RCR1 protein in a recipient plant to obtain a transgenic or gene-edited plant, wherein the transgenic or gene-edited plant has a disease resistance lower than that of the recipient plant;
the RCR1 protein is as defined in N1) or N2) or N3) or N4):
N1) a protein having an amino acid sequence of Sequence 2;
N2) a fusion protein obtained by attaching a tag to the N-terminus and/or C-terminus of the protein of Sequence 2;
N3) a plant disease resistance-related protein derived from the amino acid sequence of Sequence 2 by substitution, and/or deletion, and/or addition of one or more amino acid residues;
N4) a plant disease resistance-related protein having 75% sequence identity to Sequence 2 and retaining plant disease resistance-related activity.

18. The method according to claim 17, wherein: the disease resistance is resistance to *Plasmodiophora brassicae.*

19. The method according to claim 17 or 18, wherein: the plant is as defined in M1) or M2) or M3) or M4):
M1) a monocotyledonous or dicotyledonous plant;
M2) a Brassicaceae plant;
M3) an *Arabidopsis thaliana;*
M4) a *Brassica napus.*

20. A transgenic plant or gene-edited plant prepared by the method according to any one of claims 17-19.
